# NEW EUROPEAN PATENT SPECIFICATION

(11) **EP 0 416 892 B2**
(45) Date of publication and mention of the opposition decision: **21.10.1998**
(45) Mention of the grant of the patent: 09.11.1994
(21) Application number: 90309708.7
(22) Date of filing: 05.09.1990
(51) Int. Cl.: A23K 1/00, A61K 35/74

(54) **Agents for the prevention and treatment of diarrhoea**
Mittel für die Verhütung und Behandlung von Diarrhöe
Agents pour la prévention et le traitement de la diarrhée

(30) Priority: 05.09.1989 JP 229653/89
(43) Date of publication of application: 13.03.1991
(73) Proprietor: AJINOMOTO CO., INC., Tokyo 104 (JP)
(72) Inventor: Onishi, Norimasa, Central Research Lab. of, Kawasaki-shi, Kanagawa-ken (JP); Yamashiro, Akihiro, Central Research Lab. of, Kawasaki-shi, Kanagawa-ken (JP)
(74) Representative: Armitage, Ian Michael

(56) References cited:
- EP-A- 0 271 364
- EP-A- 0 279 618
- GB-A- 2 221 829
- Abstract of JP-A-56-108717
- English translation of JP-A-62-265231
- Microbial Cell Walls and Membranes, H.J. Rogers et al, 1980, pp. 192-205, 464-469
- Bergey's Manual of Systematic Bacteriology, vol. 2, 1986, pp. 1426, 1432
- Stedman's Medical dictionary, 25. ed., 1990, pp. 1486-1487
- Zeitschrift für Immunitätsforschung, 159(1), 1975, pp. 302, 303, 306-308

## Description

The present invention relates to agents for the prevention and treatment of diarrhoea. In particular, the present invention relates to agents for the prevention and treatment of diarrhoea in, for example, livestock, poultry, pet animals, etc.

White diarrhoea or diarrhoea in a lactation period is currently one of the most serious problems for swine breeders. About 45 to 70% of swine of 2 to 9 weeks age which are being suckled and weaned, suffer from white diarrhoea or diarrhoea. This is a serious problem in any swine breeding unit, and causes serious financial losses owing to the loss of piglets, veterinary costs etc.

The problem is not restricted only to those countries with less advanced husbandry techniques, as it is also recognised in more developed countries. In order to prevent and treat this disease, antibiotics such as sulfa drugs or the like are generally used, but in recent years, the use of antibiotics has been restricted, because of the appearance of resistant bacteria and because of the internal persistence of chemicals.

For the prevention and treatment of white diarrhoea/diarrhoea in livestock or poultry, live bacterial compositions have been developed as alternative agents to antibiotics. In this form of treatment, useful live bacteria are directly administered to livestock or poultry, whereby the administered bacteria are retained in the intestine of livestock and compete with, and therefore antagonise, the harmful enterotoxic bacteria, e.g., Escherichia coli, to eliminate the enterotoxic bacteria, during the passage of the administered bacteria through the intestine. As a result, the enterobacterial microflora is improved, so that diarrhoea is prevented and treated. Therefore, in order to be effective, it is necessary that the bacteria administered are alive. Thus, for purposes of enhancing the physicochemical stability of the live bacterial composition in animal feedstuffs and in the digestive tract, there are examples reported of using spore forming bacteria [Veterinarian and Stockbreeders' News, No. 695, page 343, 1979; Journal of Japanese Veterinary Association, 30, 645 (1977)], and of utilising multi-drug resistant bacteria, taking into account the use of bacteria in combination with antibiotics as feed additives [Bifidobacteria Microflora, 4, 15 (1985)].

However, few of the known live bacterial agents are very stable. In addition, the effect exerted by those known live bacterial agents is very slow. This is because they are thought to exhibit their effects by virtue of their setting up in competition against the harmful bacterial colony.

EP-A-0 279 618 (Porter) discloses a probiotic-type product for improving the rate of growth and feed conversion efficiency in young animals, which product comprises killed cells of lactobacilli, streptococci or b.subtilis.

Another composition for the prevention and treatment of white diarrhoea/diarrhoea in livestock or poultry has been proposed, which comprises as an effective ingredient, the enzymatically digested product of the cell wall of Bifidobacterium thermophilum. This species is considered to be an intestinal bacteria with beneficial effects for livestock (cf. Japanese Patent Application Laid-Open No. 56-108717). It is also reported that among the cell wall degradation products of bacteria belonging to the genus Bifidobacterium, peptide glycan is the effective ingredient (cf. Japanese Patent Application Laid-Open No. 62-265231). Glycan is a component of all bacterial cell walls and the general structure is common to all species. However, there are significant variations in the precise structure of glycan between different microbial species.

The mechanism of the activity of this composition is quite dissimilar to that of the conventional live bacterial agents described above, since the activity is considered to be effected by the activation of the immune system. In this way, the ability of livestock to withstand infection is enhanced. The reported methods comprise culturing useful bacteria inherently present in the intestine of livestock, isolating the effective ingredient and administering the effective ingredient to the host. Bifidobacterium used in this method is a strict anaerobe, which requires an oxygen-free incubation environment to support bacterial cell growth. This requirement makes incubation difficult and complicated. In addition, expensive raw materials such as vitamins, etc. are required for the growth of Bifidobacterium. Nevertheless, cell yield is poor, resulting in high production costs.

The present invention seeks to ameliorate any of the above or other problems. For example, the present invention seeks to provide agents for the prevention and treatment of white diarrhoea/ diarrhoea by activation of the immune system in for example livestock, and which unlike antibiotics, are free of the problems related drug resistant bacteria or the internal persistence of drugs, and which are more highly effective than live bacterial agents. Known agents which function, by activating the immune system rely on bacteria which are difficult to handle, and which are difficult to grow giving poor yields.

Accordingly, the present invention seeks to develop agents for the prevention and treatment of diarrhoea in for example livestock, poultry, pet animals etc., which can be readily produced on an industrial scale, which can be easily handled and which are efficient in activating the immune system.

In view of the foregoing situation, the present inventors have made extensive investigations and as a result, the present inventors have found that substances containing cell wall components of bacteria belonging to the genera Brevibacterium, Corynebacterium, or Escherichia, which are not intestinal bacteria derived from for example livestock, but which can grow under aerobic conditions, are effective in preventing and treating white diarrhoea or diarrhoea in for example, livestock, poultry, pet animals, etc.

The present invention provides the use of one or more of:
killed bacterial cells belonging to one or more of the genera: Brevibacterium; Corynebacterium; and Escherichia;
a mechanical homogenate of bacterial cells belonging to one or more of the genera: Brevibacterium; Corynebacterium; and Escherichia;
an enzyme homogenate as obtainable by enzymatic degradation, with an enzyme capable of degrading cell wall, of bacterial cells or of a mechanical homogenate thereof, said cells belonging to one or more of the genera: Brevibacterium; Corynebacterium; and Escherichia;
and/or an isolated fraction obtainable by fractionation of a said homogenate and containing a component of bacterial cell wall of bacterial cells belonging to one or more of the genera Brevibacterium; Corynebacterium; and Escherichia;
   for the manufacture of an agent which may be in the form of a pharmaceutical composition or a feed additive for the prevention and treatment of diarrhoea. The composition or additive may comprise as the active ingredient any of the compounds cited above, namely: killed bacterial cells; a homogenate of bacterial cells; or a fraction containing a component of a bacterial cell wall; or which comprises as the active ingredient any one of the combinations of: killed bacterial cells and a homogenate of bacterial cells; killed bacterial cells and a fraction containing a component of a bacterial cell wall; a homogenate of bacterial cells and a fraction containing a component of a bacterial cell wall; or killed bacterial cells, a homogenate of bacterial cells and a fraction containing a component of a bacterial cell wall. In particular, the composition or additive may comprise as the active ingredient any one of the above identified combinations.

The bacterial cells may be non-intestinal. The bacterial cells may be aerobic. Killed cells may be obtained by sterilisation The cell homogenate may be obtained by mechanical homogenisation and/or enzymatic decomposition. The fraction may be obtained by fractionating the cell homogenate or a homogenate of a bacterial cell wall. The agent may also comprise one or more excipients.

Examples of bacteria which can be used in the agent of the present invention for the prevention and treatment of white diarrhoea/diarrhoea, include bacteria belonging to the genus Brevibacterium such as Brevibacterium lactofermentum ATCC 13869; bacteria belonging to the genus Corynebacterium such as Corynebacterium glutamicum ATCC 13060; bacteria belonging to the genus Escherichia such as Escherichia coli ATCC 8739.

For incubation of these bacteria, any nutrient source which the bacteria can utilise can be used. For example, conventional media appropriately supplemented with carbohydrates such as glucose, or sucrose; alcohols such as ethanol, or glycerol; organic acids such as acetic acid, or priopionic acid; carbon sources such as soybean oil or a mixture of different carbon sources; nitrogen-containing inorganic or organic nutrient sources such as yeast extract, peptone meat extract, corn steep liquor, ammonium sulphate, or ammonia; inorganic nutrient sources such as phosphates, magnesium, iron, manganese, or potassium; vitamins such as biotin, or thiamine. Incubation may be carried out in a nutrient medium within a pH range of 4.0 to 9.5 at 20 to 40°C for 12 hours to 5 days under aerobic conditions.

The cells obtained by the incubation are isolated from the medium and killed by for example heat treatment. The killed cells may be used as they are. However, it is preferable to use homogenised cells. The process of homogenisation can also be used as a method of killing the microbial cells. The cells to be homogenised may be alive cells and/or killed cells. A method for homogenisation may be a mechanical method and/or a method utilising an enzyme. In a suitable mechanical method, the cells are homogenised using, e.g., a French press. under a pressure of about 800 to 2000 kg/cm² Alternatively, the cells may also be homogenised with an ultrasonic homogeniser. In the case of homogenising the bacterial cells using an enzyme, the cultured cells or mechanical homogenate of the cultured cells are suspended in, for example, physiological saline, and an enzyme for lysing the cell wall is added to the suspension to degrade the cell wall. The enzyme used for this purpose may be any enzyme capable of degrading cell wall. Representative examples of the enzyme are lysozyme, and protease. Conditions for treatment with enzyme are set forth following known methods. In either the mechanical method or the enzyme method, it is preferable that the amount of cell homogenisation is 20% or more, preferably about 60%. The amount of homogenisation can be determined in terms of the reduction in turbidity of a suspension of microbial cells at a wavelength of 660 nm. The percentages given above, relate to the turbidity decrease in a suspension of microbial cells at a wavelength of 660 nm and not to the percentage of cells killed. For example, when the cell homogenisation is 20% substantially all microbial cells are killed. In order to increase the amount of homogenisation, it is preferable to use the mechanical method and the enzyme method in combination.

The cell homogenate may also be fractionated and the isolated fraction containing cell wall components may be used. Fractionation may be effected by simply centrifuging the cell homogenate to remove insoluble matters. Furthermore, known methods for fractionating proteins based on, for example molecular weight using methods such as ultrafiltration, or gel filtration, may also be utilised.

The use of the composition of the present invention for the prevention and treatment of diarrhoea will now be described. The composition in the form of prepared cells, homogenates thereof or fractions containing the cell wall components (separately or in combination) may be orally given to the subject (for example, livestock, poultry, pet animals,) in the form of liquid. If necessary and desired, the composition may be dried to a powdery form, which is added to, for example, animal feedstuffs. The composition may be administered as a treatment. However, for the prevention and treatment of white diarrhoea/diarrhoea in young animals being suckled, the composition can be given consecutively for 1 to 2 weeks from birth, thereby enhancing its beneficial effect. A daily dose is about 10 mg to about 50 g, preferably about 100 mg to about 2 g, on a dry weight basis. During the weaning period in pigs or poultry, the composition may be added to the feed in an amount comprising 0.01 to 5%, preferably 0.05 to 1%, on a dry weight basis.

In the present invention, livestock includes for example, swine. cow, horse, goat, sheep; poultry includes fowls such as chicken; and pets includes for example, dogs and cats.

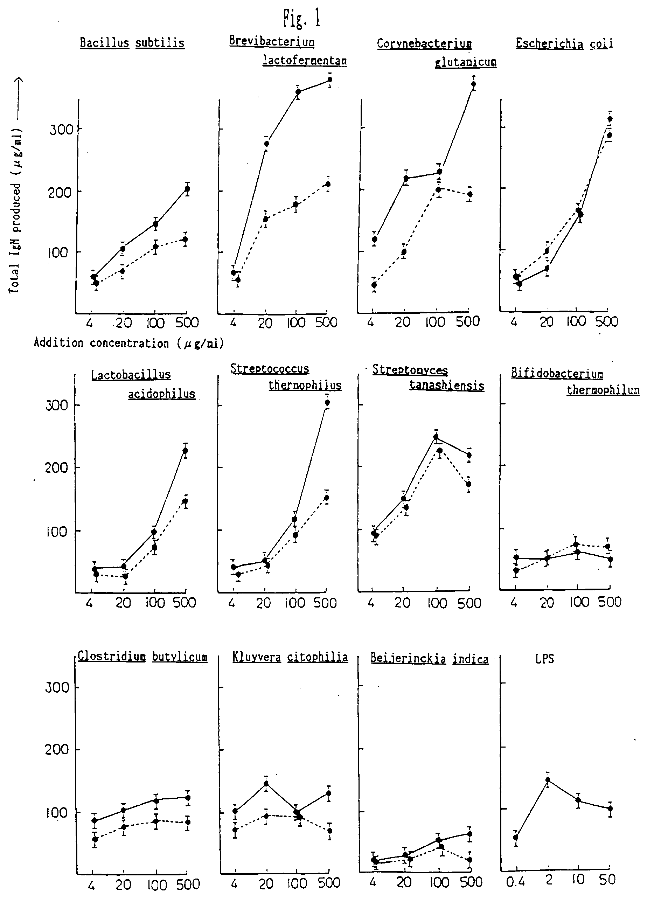

The invention will now be described in more detail with reference to the following examples provided by way of illustration only, and not by way of limitation and with reference to figure 1 which shows graphs of the relationship between the killed cells from each genus and their enzyme degradation products, and mouse IgM antibody production.

In the examples which follow Bacillus subtilis, Lactobacillus acidophilus, Streptococcus thermophilus and Streptomyces tanashiensis are used as comparative examples.

### Example 1

### Growth of Bacterial Cells

Flasks of 500 ml volume were charged with 50 ml of medium (pH 7) containing 1.0 g/dl of glucose, 1.0 g/dl of yeast extract, 1.0 g/dl of peptone, 0.5 g/dl of (NH₄)₂SO₄, 0.3 g/dl of K₂HPO₄, 0.3 g/dl of K₂HPO₄, 0.1 g/dl of KH₂PO₄ and 0.05 g/dl of MgSO₄.7H₂O followed by sterilisation at 115°C for 15 minutes. Bacillus subtilis ATCC 13952, Brevibacterium lactofermentum ATCC 13869, Corynebacterium glutamicum ATCC 13060, Escherichia coli ATCC 8739, Lactobacillus acidophilus ATCC 4356, Streptococcus thermophilus ATCC 19987, Streptomyces tanashiensis ATCC 15238, were precultured (separately) in bouillon agar medium at 30°C for one day. A loopful of each culture was then inoculated on separate flasks of the sterilised medium, followed by shake culture at 30°C for 24 hours. After completion of the culture, each medium was centrifuged to collect the cells. The respective cells were suspended in a volume of physiological saline equivalent to that of the medium, followed by a heat treatment at 100°C for 10 minutes. Each suspension was again centrifuged to collect the cells.

For control, Bifidobacterium thermophilum ATCC 25525 (derived from swine intestine) and Clostridium butylicum (derived from human intestine) were used as strict anaerobes. Flasks of 300 ml volume were charged with 280 ml of medium (pH 7.0) containing 1.0 g/dl of glucose, 1.0 g/dl of yeast extract, 1.0 g/dl of peptone, 0.02 g/dl of MgSO₄.7H₂O, 0.05 g/dl of Tween 80, 0.2 g/dl of ammonium acetate, 10% tomato juice filtrate and 1.0 g/dl of CaCO₃ followed by sterilisation at 115°C for 15 minutes. The control bacteria were subjected to stab culture in bouillon agar medium and then inoculated on separate flasks of the sterilised medium, followed by culturing at 37°C for 2 days under anaerobic conditions. After completion of the culture, each medium was centrifuged to collect the cells. The respective cells were suspended in a volume of physiological saline equivalent to that of the medium. The suspension was heat treated at 100°C for 10 minutes and again centrifuged to collect the cells.

Weight of wet cake obtained per 100 ml of medium after the culture, is shown in Table 1.

**Table 1**

| | Wt of Wet Cake g/100 ml |
|---|---|
| Bacillus subtilis ATCC 13952 | 4.2 |
| Brevibacterium lactofermentum ATCC 13869 | 5.6 |
| Cornynebacterium glutamicum ATCC 13060 | 5.3 |
| Escherichia coli ATCC 8739 | 4.5 |
| Lactobacillus acidophilus ATCC 4356 | 0.8 |
| Streptococcus thermophilus ATCC 19987 | 0.5 |
| Streptomyces tanashiensis ATCC 15238 | 2.1 |
| Bifidobacterium thermophilum ATCC 25525 | 0.2 |
| Clostridium butylicum | 0.3 |

### Example 2

### Homogenisation

The respective cells (wet cakes) prepared in example 1 were each suspended in 25 mM phosphate buffer (pH 7.0) in 10 wt %, respectively. Each cell suspension was put in a separate stainless steel bottle (50 ml volume) and treated for 15 minutes at an oscillation frequency of 20 kHz and an output of 200 W using an ultrasonic homogeniser (Model UR-200 P, manufactured by Tomy Seiko Co., Ltd.). After the treatment, each homogenate was further centrifuged to fractionate a fraction containing the cell wall components.

### Example 3

### Enzyme Degradation Product

To separate aliquots of 25 mM phosphate buffer (pH 7.0) each containing as a solid content 10 wt % of a different mechanical cell homogenate prepared as described in example 2 above, were added 0.01 wt % of albumen lysozyme (manufactured by Sigma Co. ) and 0.02 wt % of actinase (manufactured by Kaken Pharmaceutical Co., Ltd., 7000 units). Each mixture was treated at 37°C for 12 hours and then heat-treated at 100°C for 2 minutes to inactivate the enzyme.

### Example 4

### Effect of Immunological Activation Using Mouse Spleen Cells

RPMI 1640 medium was supplemented with 10% immobilised fetal calf serum and 5 x 10 ⁻⁵ M 2-mercaptoethanol, and then filtered and sterilised. Spleen cells prepared from the spleen of a DBA/2 strain female mouse of 10 weeks old were suspended in the sterilised medium at a concentration of 2.5 x 10⁶ cells/ml. The cells and enzyme degradation product prepared in examples 1 and 3 or cells of Kluyvera citophilia IFO 8193 and Beijerinckia indica ATCC 9037 and the enzyme degradation product prepared in a manner similar to examples 1 and 3 were added to the respective suspensions in the respective concentrations, followed by culturing at 37°C for 4 days in a 5% CO₂ incubator. After the culture, the supernatants were diluted to 1000-fold with 0.01 M Tris-hydrochloride buffer (pH 8.1) containing 1% bovine serum albumin. Thereafter, the total amount of mouse IgM produced in each medium was measured by ELISA to determine the effect on immunological activation in each system. As a standard substance for assaying the effect on immunological activation, lipopolysaccharide (LPS) derived from pathogenic Escherichia coli was used. The results are shown in figure 1.

Figure 1 shows the relationship between the killed cells from each genus (broken lines) and the enzyme degradation products thereof (solid lines) and mouse IgM antibody production. In the figure, the abscissa indicates the concentration (µg/ml) of killed bacteria cells added, and the ordinate indicates the concentration (µg/ml) of total mouse IgM produced in the culture broth of spleen culture cells.

As is clear from figure 1, the antibody productivity of Brevibacterium lactofermentum ATCC 13869, Corynebacterium glutamicum ATCC 13060, Escherichia coli ATCC 8739, according to the present invention, is higher than that of Bifidobacterium thermophilum or Clostridium butylicum which is used as a strict anaerobe derived from the intestine of livestock, and higher than that of Kluyvera citophilia and Beijerinckia indica which is used as an aerobe, in the control groups.

In addition, a tendency for the antibody productivity of the enzyme degradation products according to the present invention to be higher than the killed cells is noted. The enzyme degradation products all show a high activity exceeding the maximum value (150 g/ml) of antibody production in LPS. This indicates that the enzyme degradation products are very effective in activating the immune system. In contrast, no activity greater than that shown by LPS was noted in the control groups. The gradients of the slopes of figure 1, show that it is impossible to expect potentiated antibody production in the control groups, even if the concentration of agents added is increased.

### Example 5

### Effect on White Diarrhoea or Diarrhoea in Piglets During Lactation

Sixty three (63) piglets being suckled were divided into 9 groups, each group comprising 7 piglets. One group was a control (non-administered group). In the other eight groups, a suspension prepared daily by suspending in 3 ml of water, 200 mg of dry powders of the enzyme degradation products of the various bacterial cells (prepared as described in example 4), was orally administered once a day for 7 consecutive days from birth.

For 20 days from day 8 to day 27 from birth, the piglets were checked for white diarrhoea or diarrhoea. As shown in Table 2 the incidence of white diarrhoea or diarrhoea was low and the average gain in body weight was high, in any of the groups administered with the enzyme degradation products of bacteria as provided by the present invention, as compared to the control group and to the groups administered with the enzyme degradation products of Bifidobacterium thermophilum.

**Table 2**

| Administered Bacteria | Total Days Observed*¹ | Total Days with White Diarrhoea or Diarrhoea*² | Body Weight Increment on day 27 (kg/pig) |
|---|---|---|---|
| Control (none) | 132*³ | 84 | 4.2 |
| Bacillus subtilis ATCC 13952 | 140 | 32 | 6.5 |
| Brevibacterium lactofermentum ATCC 13869 | 140 | 25 | 6.8 |
| Corynebacterium glutamicum ATCC 13060 | 140 | 28 | 6.5 |
| Escherichia coli ATCC 8739 | 140 | 23 | 6.3 |
| Lactobacillus acidophilus ATCC 4356 | 140 | 46 | 5.9 |
| Streptococcus thermophilus ATCC 19987 | 140 | 36 | 6.1 |
| Streptomyces tanashiensis ATCC 15238 | 124*⁴ | 50 | 5.0 |
| Bifidobacterium thermophilum ATCC 25525 | 140 | 58 | 4.8 |

| | | | |
|---|---|---|---|
| *1 The number (7) of animals tested x the number (20 days) of days observed. | | | |
| *2 Total days when white diarrhoea or diarrhoea was observed during the total number of days for observation. | | | |
| *3, 4 One pig in each group was dead. | | | |

### Example 6

### Effect on White Diarrhoea or Diarrhoea in Piglets During Lactation

Twenty eight (28) piglets being suckled were divided into 4 groups, each group comprising 7 piglets. One group was a control (non-administered group). In the other three groups (administered groups), suspensions prepared daily by suspending in 3 ml of water, 200 mg of dry powders of: i) the heat-treated cells of Brevibacterium lactofermentum ATCC 13869; ii) its mechanical homogenate; and iii) its enzyme degradation products and fraction thereof prepared as described in examples 1, 2 and 3 were orally administered to the respective groups once a day for 7 consecutive days from birth.

For 20 days from day 8 to day 27 from birth, the piglets were checked for white diarrhoea or diarrhoea. As shown in Table 3, the incidence of white diarrhoea or diarrhoea was low and the average gain in body weight was high in any of the groups receiving treatment according to the present invention, as compared to the control group.

In the experimental groups, the preventative effect on the occurrence of white diarrhoea or diarrhoea, was greater in the groups administered with the mechanical homogenate and its enzyme degradation products, than in the group administered with the heat-treated cells.

**Table 3**

| Administered Bacteria | Total Days Observed*¹ | Total Days with White Diarrhoea or Diarrhoea*² | Body Weight Increment on day 27 (kg/pig) |
|---|---|---|---|
| Control (none) | 132*³ | 84 | 4.2 |
| Heat-treated cells | 140 | 46 | 5.4 |
| Mechanical homogenate of cells | 140 | 30 | 6.7 |
| Enzyme degradation products of cells | 140 | 25 | 6.8 |

| | | | |
|---|---|---|---|
| *1 The number (7) of animals tested x the number (20 days) of days observed. | | | |
| *2 Total days when white diarrhoea or diarrhoea was observed during the total number of days for observation. | | | |
| *3 One pig in this group was dead. | | | |

### Example 7

### Feeding Test of Calves by Addition to Feed

Twelve (12) male calves of the Holstein species, one week after birth, were divided into 3 groups. One group was the control (non-administered) group. In the other two groups, the calf feed fed to the calves during weaning had added to it 0.1% (for one group) and 1% (for the other group) of dry powders of the enzyme degradation products of Brevibacterium lactofermentum ATCC 13869 prepared according to example 4 (administered groups). The calves were suckled for 3 weeks. During the period, the incidence of diarrhoea and soft faeces was observed and the gains in body weight were measured.

As shown in Table 4, the incidence of diarrhoea and soft faeces was low and the gain in body weight was good in the administered groups, as compared to the control group. Furthermore, in the administered groups, the 1% administered group gave somewhat better results than the 0.1% administered group.

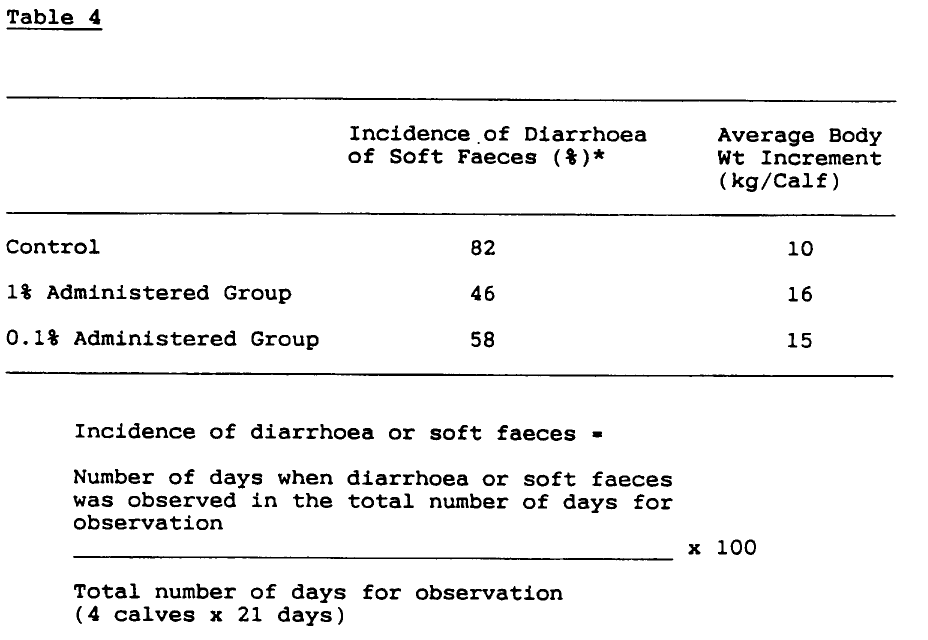

## Claims

1. The use, of any one or more of:
killed bacterial cells;
a mechanical homogenate of bacterial cells;
an enzyme homogenate as obtainable by enzymatic degradation, with an enzyme capable of degrading cell wall, of bacterial cells, or of a mechanical homogenate thereof; and/or
an isolated fraction, obtainable by fractionation of a said homogenate, and containing a component of bacterial cell wall of bacterial cells;
said bacterial cells belonging to any one or more of the genera: Brevibacterium; Corynebacterium; and Escherichia for the manufacture of a pharmaceutical composition or a feed additive, for the prevention and treatment of diarrhoea,.

2. The use according to claim 1 wherein the composition or additive comprises as the active ingredient any one of:
killed bacterial cells;
a said homogenate of bacterial cells; or
a said fraction containing a component of a bacterial cell wall;
or which comprises as the active ingredient any one of the combinations of:
killed bacterial cells and a said homogenate of bacterial cells;
killed bacterial cells and a said fraction containing a component of a bacterial cell wall;
a said homogenate of bacterial cells and a said fraction containing a component of a bacterial cell wall;
or
killed bacterial cells, a said homogenate of bacterial cells and a said fraction containing a component of a bacterial cell wall.

3. The use according to claim 1 or claim 2 wherein said bacterial cells are:
Brevibacterium lactofermentum ATCC 13869;
Corynebacterium glutamicum ATCC 13060;
Escherichia coli ATCC 8739;

4. The use according to any one of the preceding claims wherein said bacterial cells are non-intestinal.

5. The use according to any one of the preceding claims wherein said bacterial cells are aerobic.

6. The use according to any one of the preceding claims wherein the composition or additive is in the form of a liquid or dry powder.

## Patentansprüche

1. Die Verwendung einer oder mehrerer der folgenden Komponenten:
abgetötete Bakterienzellen,
ein mechanisches Homogenat von Bakterienzellen,
ein Enzymhomogenat, das durch enzymatischen Abbau von Bakterienzellen oder eines mechanischen Homogenats davon mit einem zum Zellwandabbau befähigten Enzym erhältlich ist, und/oder
eine isolierte Fraktion, die durch Fraktionierung eines der Homogenate erhältlich ist und eine Komponente einer Bakterienzellwand von Bakterienzellen enthält;
wobei die Bakterienzellen einer oder mehreren der Gattungen Brevibacterium, Corynebacterium und Escherichia angehören;
zur Herstellung einer Arzneimittelzusammensetzung oder eines Nahrungsmittelzusatzes zur Vorbeugung und Behandlung von Diarrhöe.

2. Verwendung gemäß Anspruch 1, wobei die Zusammensetzung oder der Zusatz eine der folgenden Komponenten als den aktiven Inhaltsstoff enthält:
abgetötete Bakterienzellen,
eines der Homogenate von Bakterienzellen oder
eine der Fraktionen, die eine Komponente einer Bakterienzellwand enthält;
oder wobei die Zusammensetzung oder der Zusatz eine der folgenden Kombinationen als den aktiven Inhaltsstoff enthält:
abgetötete Bakterienzellen und eines der Homogenate von Bakterienzellen,
abgetötete Bakterienzellen und eine der Fraktionen, die eine Komponente einer Bakterienzellwand enthält,
eines der Homogenate von Bakterienzellen und eine der Fraktionen, die eine Komponente einer Bakterienzellwand enthält, oder
abgetötete Bakterienzellen, eines der Homogenate von Bakterienzellen und eine der Fraktionen, die eine Komponente einer Bakterienzellwand enthält.

3. Verwendung gemäß Anspruch 1 oder 2, wobei die Bakterienzellen die folgenden sind:
Brevibacterium lactofermentum ATCC 13869;
Corynebacterium glutamicum ATCC 13060;
Escherichia coli ATCC 8739.

4. Verwendung gemäß einem der vorstehenden Ansprüche, wobei die Bakterienzellen nicht-intestinal sind.

5. Verwendung gemäß einem der vorstehenden Ansprüche, wobei die Bakterienzellen aerob sind.

6. Verwendung gemäß einem der vorstehenden Ansprüche, wobei die Zusammensetzung oder der Zusatz in der Form einer Flüssigkeit oder eines trockenen Pulvers ist.

## Revendications

1. Utilisation d'un ou plusieurs des produits suivants:
des cellules bactériennes tuées;
un homogénat mécanique de cellules bactériennes;
un homogénat enzymatique tel qu'il peut être obtenu par dégradation enzymatique, avec une enzyme capable de dégrader la paroi cellulaire, de cellules bactériennes ou d'un homogénat mécanique de ces cellules; et/ou
une fraction isolée, pouvant être obtenue par fractionnement d'un de ces homogénats, et contenant un constituant de paroi cellulaire bactérienne de cellules bactériennes;
lesdites cellules bactériennes appartenant à un ou plusieurs des genres: *Brevibacterium; Corynebacterium*; et *Escherichia*, pour la préparation d'une composition pharmaceutique ou d'un additif alimentaire pour la prévention et le traitement de la diarrhée.

2. Utilisation selon la revendication 1, dans laquelle la composition ou l'additif comprend comme ingrédient actif l'un quelconque des produits suivants:
des cellules bactériennes tuées;
l'un desdits homogénats de cellules bactériennes;
ladite fraction contenant un constituant de paroi cellulaire bactérienne; ou qui comprend comme ingrédient actif l'une quelconque des combinaisons constituées par:
des cellules bactériennes tuées et l'un desdits homogénats de cellules bactériennes;
des cellules bactériennes tuées et ladite fraction contenant un constituant de paroi cellulaire bactérienne;
l'un desdits homogénats de cellules bactériennes et ladite fraction contenant un constituant de paroi cellulaire bactérienne; ou
des cellules bactériennes tuées, l'un desdits homogénats de cellules bactériennes et ladite fraction contenant un constituant de paroi cellulaire bactérienne.

3. Utilisation selon la revendication 1 ou la revendication 2, dans laquelle lesdites cellules bactériennes sont:
*Brevibacterium lactofermentum* ATCC 13869;
*Corynebacterium glutamicum* ATCC 13060;
*Escherichia coli* ATCC 8739.

4. Utilisation selon l'une quelconque des revendications précédentes, dans laquelle lesdites cellules bactériennes sont non intestinales.

5. Utilisation selon l'une quelconque des revendications précédentes, dans laquelle lesdites cellules bactériennes sont aérobies.

6. Utilisation selon l'une quelconque des revendications précédentes, dans laquelle la composition ou l'additif est sous forme d'un liquide ou d'une poudre sèche.
